Europäisches Patentamt

European Patent Office

Office européen des brevets

(11) Veröffentlichungsnummer: 0 248 103
A1

(12) **EUROPÄISCHE PATENTANMELDUNG**

(21) Anmeldenummer: 86107721.2

(51) Int. Cl.⁴: A61B 5/03

(22) Anmeldetag: 06.06.86

(43) Veröffentlichungstag der Anmeldung:
09.12.87 Patentblatt 87/50

(84) Benannte Vertragsstaaten:
AT BE CH DE FR GB LI NL

(71) Anmelder: Hellige GmbH
Postfach 728 Heinrich-von-Stephan-Strasse 4
D-7800 Freiburg im Breisgau(DE)

(72) Erfinder: Ullrich, Georg J., Dipl.-Physiker
Mettackerweg 84
D-7800 Freiburg im Breisgau(DE)
Erfinder: Pomorin, Karl-Heinz
Ringstrasse 8
D-7801 Stegen(DE)
Erfinder: Kronberg, Harald, Dr.-Physiker
Mülhauserstrasse 6
D-7813 Staufen(DE)
Erfinder: Bramm, Guenter, Dipl.-Ing.
Otto-Walle-Strasse 14,
D-6676 Mandelbachtal 3,(DE)
Erfinder: Koschke Peter, Dipl.-Ing.
Dettendorf 4 1/4,
D-8201 Bad Feilnbach,(DE)
Erfinder: Novak, Pavel, Dr.-Ing.
Schleissheimer-Strasse 44,
D-8000 Muenchen 2,(DE)
Erfinder: Gaab, Michael, Prof. Dr.
Senator-Bauer-Strasse 36,
D-3000 Hannover 61,(DE)

(74) Vertreter: Patentanwälte TER MEER - MÜLLER
- STEINMEISTER
Mauerkircherstrasse 45
D-8000 München 80(DE)

(54) **Applikationsvorrichtung zur auswechselbaren Implantation von Biosensoren in die Schädelkalotte.**

(57) Die Applikationsvorrichtung zur auswechselbaren Implantation von Biosensoren in die Schädelkalotte besteht im wesentlichen aus zwei ineinander steckbaren Hülsen, einer Federbeinhülse (4) mit außenseitigen Rastnocken (13), welche die Innenumrandung eines Bohrlochs (2) im Schädelknochen (1) untergreifen, und aus einer in die Federbeinhülse (4) einzusteckenden Spreizhülse (9). Der axiale Bezug und Toleranzausgleich wird durch ein als Kautschukring ausgeführtes Federelement (8) gewährleistet. Der Meßaufnehmer (10) läßt sich problemlos mit Reib-oder eng toleriertem Gleitsitz in die Spreizhülse (9) einsetzen, wobei der axiale Bezug durch wenigstens eine Paßfläche gewährleistet ist.

Fig. 3

## Applikationsvorrichtung zur auswechselbaren Implantation von Biosensoren in die Schädekalotte

Die Erfindung betrifft eine Applikationsvorrichtung zur auswechselbaren Implantation von Biosensoren in die Schädelkalotte gemäß dem Oberbegriff des Patentanspruchs 1.

Um den Schädelinnendruck messen zu können, ist es bisher üblich, ein Loch von etwa 11 mm Durchmesser in den Schädelknochen zu bohren und ein Gewinde einzuschneiden, um einen Drucksensor tiefengerecht und mit koplanar zur Dura mater (harten Hirnhaut, im folgenden kurz "Dura") ausgerichteter Meßmembran justieren zu können. Dabei muß genau nachgemessen werden, wie tief das Gewinde geschnitten werden kann. Beim Gewindeschneiden selbst, aber auch beim Schraubjustieren des Sensors, wirken reltiv hohe, insbesondere radiale Kräfte, was zu einem Zurückweichen des Knochenmaterials führt. Die Folge ist, daß das gasamte Meßsystem auch dann, wenn eine sogenannte Adapterhülse verwendet wird, die mit Gewinde in den Knochen eingesetzt wird, nach einiger Zeit zu wackeln beginnt. Es muß dann immer wieder nachjustiert werden, um das unbedingte Erfordernis für eine genaue Messung einhalten zu können, nämlich die koplanar-epidurale Ausrichtung der Meßmembran.

Ein weiteres Problem bei bisher üblichen Druckmeßverfahren im Schädelinneren ergibt sich durch das starke Verkleben der Applikationshülsen im Knochen. Müssen die Applikationshülsen nach ausreichendem Meßzeitraum wieder explantiert werden, so tritt es nicht selten auf, daß ein Teil des Knochens herausgebrochen oder herausgerissen wird. Weiterhin zeigt sich in der Praxis, daß auch das Gewindeschneiden im Schädelknochem zum Einsetzten der Applikationshülse eine große Erfahrung des Gehirnchirurgen verlangt. Trotz großer Erfahrung treten immer wieder Probleme mit dem Verkanten des Meßaufnehmers auf.

Eine Verbesserung gegenüber dem herkömmlichen Verfahren mit eingeschraubter Applikatioshülse wurde mit dem in der DE-A 3 135 511 beschriebenen Drucksensor erreicht. Dieser Drucksensor wird so appliziert, daß zunächst nach dem Aufschneiden der Kopfschwarte mittels eines Stufentrepans ein definiert abgestuftes Loch in den Schädelknochen gebohrt wird, in das die Applikationshülse in Form einer mit nach außen spreizenden Federbeinen versehenen Spreizhülse eingesetzt wird, die im oberen Bereich einen flanschartig nach außen ragenden verbreiterten Abschnitt aufweist, der im Zusammenwirken mit der Abstufung im gebohrten Loch den Tiefenbezug für das Einsetzen des Drucksensors gewährleisten soll. die bekannte Spreizhülse (vgl. insbesondere Fig. 2 der DE-A 3 135 511) ist am unteren Ende ihrer Federbeine mit außenseitigen Rastnocken versehen, welche bei eingesetzter Spreizhülse die Unterkante des Lochs im Schädelknochen untergreifen und so die Applikationshülse ortsfest fixieren. Um den Tiefenbezug zu justieren, kann ein Zwischenring auf die Spreizhülse von den freien Enden der Spreizbeine her aufgeschoben werden, der bei in das Loch im Schädelknochen eingesetzter Spreizhülse auf der Bohrungsschulter aufliegt. Zwar lassen sich mit chirurgischen Stufentrepanen im allgemeinen gut reproduzierbare abgestufte Bohrungen erzielen. Klinische Tests haben jedoch ergeben, daß die Stufenhöhe durchaus variieren kann, beispielsweise bei einer mittleren Stufenhöhe von 2,6 mm wurden Variationen zwischen 1,7 mm und 3,8 mm Stufenhöhe festgestellt. Da die Membran des Druckaufnehmers jedoch in genau definierter Tiefenlage zu justieren ist, werden bei dem bekannten Drucksensor die Variationen der Stufenhöhe durch unterschiedliche Zwischenringe ausgeglichen.

Der bekannte Drucksensor wird in die Spreizhülse eingeschraubt, die zu diesem Zweck in ihrem oberen Bereich mit einem Innengewinde versehen ist. Auch über dieses Gewinde läßt sich eine gewisse Tiefenjustage erreichen. Das Innengewinde im oberen Bereich der Spreizhülse dient außerdem zum Verankern eines Einsetz-und Auswechselwerkzeugs. Allerdings sind auch mit der bekannten Federbeinhülse insbesondere nach längerer Meßzeit beim Auswechseln Verletzungen möglich, da, wie in der DE-A 3 135 511 erwähnt, Verklebungen im unteren Bereich der Federbeine erfolgen, die beim Herausziehen der Federbeinhülse unvermeidlicherweise Verletzungen verursachen, wenn auch zweifellos geringere als bei der früher üblichen, Schraubverankerung der Applikationshülse im Schädelknochen.

Ein besonderes Problem, das sich bei dem bekannten, Schädelinnendrucksensor gemäß der genannten DE-A stellt, ergibt sich aus folgendem: Die Federbeine der zunächst nur lose in die abgestufte Bohrung des Schädelknochens eingesetzte Spreizhülse werden erst beim Einschieben des Drucksensors in die Hülse axial nach außen gedrückt, so daß die endseitigen Rastnocken die untere Umlaufkante des Bohrlochs hintergreifen können. Leider ist es dabei unvermeidlich, daß ein gewisser Druck auch auf die Meßmembran insbesondere in deren Randbereich übertragen wird. Dies ist insbesondere dann der Fall, wenn der Drucksensor auch nur geringfügig verkantet in die Spreizhülse eingeschoben wird. Für eine genaue Druckmessung an der Dura es jedoch unbedingt erforderlich; daß keinerlei andere Druckkräfte auf die Meßmembrand des Drucksensors wirken.

Ein weiteres Problem mit dem bekannten Drucksensor ergibt sich daraus, daß auch der Tiefenbezug nur relativ umständlich oder ungenau reproduzierbar ist. Durch das verwendete Halte-und Justiergewinde in der Spreizhülse muß außerdem der Sensor reltiv "kantig" gebaut werden und muß beim Einsetzen verdreht werden, was wiederum die Gefahr mit sich bringt, daß unkontrollierbare Druckkräfte an der Unterseite, also im Bereich des Membranrands, wirken, die das Meßergebnis verfälschen können. Zwar schlägt die genannte DE-A als Alternative die Verwendung eines Bajonettverschlusses vor. Dies hat aber zur Folge, daß dann Justiermittel anderer Art verwendet werden müssen. Über längere Zeit durchgeführte Versuche haben gezeigt, daß bei Verwendung eines Bejonettverschlusses unbedingt immer wieder zu prüfen ist, ob der Verschluß adäquat eingeschnappt ist. Dabei kann wiederum leicht das bereits erwähnte, befürchtete Verkanten zu Verfälschungen des Meßergebnisses führen. Wird die Spreizhülse dagegen "locker" gehalten, so tritt es nicht selten ein, daß die Hülse zu locker sitzt und sich mitdreht, statt daß der Sensor wirklich in den Bajonettverschluß einschnappt.

Der Erfindung liegt die Aufgabe zugrunde, die bekannte Applikationsvorrichtung, wie sie ausgehend von der DE-A 31 35 511 im Oberbegriff des Patentanspruchs 1 definiert ist, so zu verbessern, daß einerseits der Drucksensor problemlos eingesetzt und ausgewechselt werden kann, ohne daß unkontrollierbare Zusatzkräfte auf die Meßflächenmembran wirken. Die zu schaffende Applikationsvorrichtung soll andererseits eine einfache Justierung und stets koplanar zur Dura sich einstellende Ausrichtung der Meßflächenmembranbei genau reproduzierbarem Tiefenbezug ermöglichen. Außerdem soll das einfache Auswechseln ermöglicht werden, ohne zusätzliche Verletzungen im Bereich des Lochs im Schädelknochen zu verursachen.

Eine Applikationsvorrichtung zur auswechselbaren Implantation von Biosensoren in die Schädelkalotte gemäß der eingangs genannten Art weist erfindungsgemäß die im Kennzeichen des Patentanspruchs 1 angegebenen Merkmale auf.

Vorteilhafte Ergänzungen und Weiterbildungen des Erfindungsgedankens sind unter anderem in Unteransprüchen angegeben und in der nachfolgenden Beschreibung erläutert.

Gemäß der Erfindung wird zusätzliche zur Federbeinhülse eine dünnwandige Spreizhülse verwendet, welche die Federbeine axial auseinanderdrückt, so daß die an den Federbeinenden vorgesehenen Rastnocken die umlaufende Unterkante des Lochs im Schädelknochen untergreifen und eine sichere Verankerung der Federbeinhülse gewähleistet ist. Vorteilhafterweise wird der Tiefenausgleich durch einen Silikonfederring erreicht, der auf die Federbeinhülse aufgeschoben wird und einerseits gegen die Bohrungsschulter und andererseits gegen die flanschartig nach außen springende Abstufung der Federbeinhülse anliegt. die gesamte Applikationsvorrichtung läßt sich so als Applikationsdoppelhülse auf einfache Weise hinsichtlich des Tiefenbezugs genau und "elastisch" justieren, wobei diese elastische Justierung bei Stoß auf die Wunde die Verletzungsgefahr vermindern kann.

Abgesehen von der viel besseren Verankerung der Federbeinhülse ergibt sich mit der Einführung der Spreizhülse hinsichtlich der Genauigkeit des Meßergebnisses folgender entscheidender Vorteil gegenüber der aus der genannten DE-A bekannten Applikationsvorrichtung: Der Drucksensor läßt sich völlig verkantfrei in die Applikationsdoppelhülse einstecken und wird mit paßgenauem Reib-oder Gleitsitz einer beispielsweise als Schulter ausgebildeten Paßfläche in der radialen Abstufung der Federbeinhülse axial fixiert. Wird die Paßfläche am Sensor als Nut ausgeführt, so kann der Sensor auch in einen in die Abstufung der Federbeinhülse eingelassenen Federring eingerastet werden. Es treten keinerlei unkontrollierbare zusätzliche Druckkräfte mehr im Bereich der umlaufenden Unterkante des Drucksensors, also im Bereich der Druckmembran, auf. Der Sensor muß nicht verschraubt oder durch Bajonettverschluß gesichert werden. Es genügt ein einfaches Einschieben oder Einstecken in die Applikationsdoppelhülse. Der richtige Tiefenbezug ist automatisch hergestellt, da die Tiefenjustierung bereits beim Einsetzen der beiden Hülsen erfolgt ist. Die Sensormembran wird -senkrechte Bohrachse vorausgesetzt - automatisch koplanar zur Dura zu stehen kommen.

Die Erfindung und vorteilhafte Einzelheiten werden nachfolgend unter Bezug auf die Zeichnung anhand eines Ausführungsbeispiels näher erläutert. Es zeigen:

Fig. 1 einen Abschnitt eines Schädelknochens, in dem eine abgestufte Bohrung mittels eines Stufentrepans in an sich bekannter Weise eingebracht wurde;

Fig. 2 das Einsetzen der Federbeinhülse in die Bohrung;

Fig. 3 das Einführung der Spreizhülse bei bereits eingesetzter Federbeinülse;

Fig. 4 das Einschieben des Meßaufnehmers; und

Fig. 5 die gesamte Meßvorrichtung bei Verwendung eines intrakraniellen Druckaufnehmers.

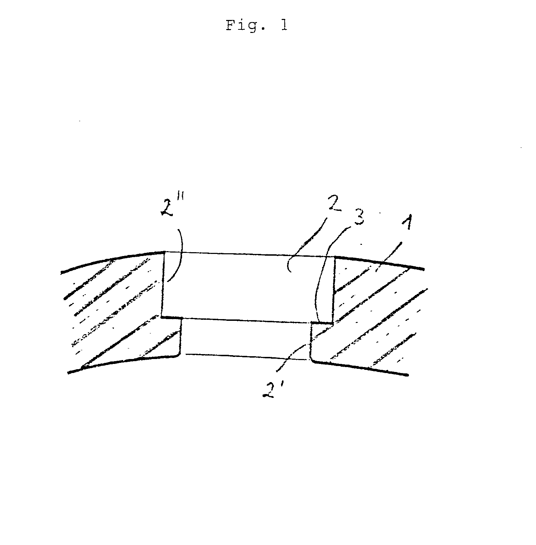

Fig. 1 verdeutlicht die Ausgangssituaiton vor dem Einsetzen der erfindungsgemäßen Applikationsvorrichtung. Vom Chirurgen wurde mittels eines bekannten Stufentrepans in einen Schädelknochen 1 ein abgestuftes Loch 2 mit einem näher an der Dura liegenden inneren engen Durchmesserbe reich 2' und einem erweiterten äußeren Durchmesser 2'' eingebracht. Eine Bohrschulter ist mit Bezugshinweise 3 angegeben.

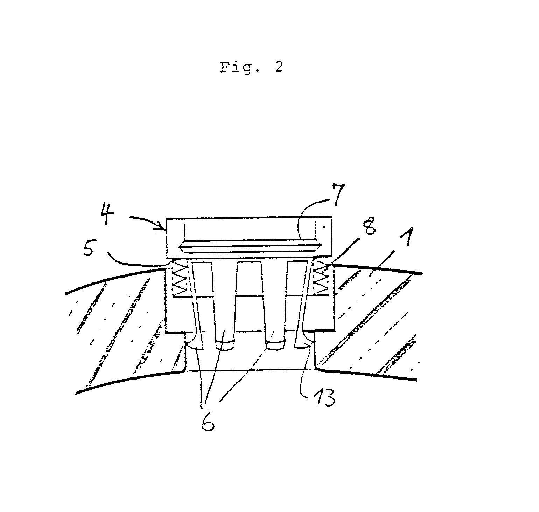

Wie die Fig. 2 zeigt, wird zunächst eine Federbeinhülse 4 mit einer Mehrzahl von radial nach innen abgeschrägt stehenden Federbeinen 6 in die Bohrung 2 eingesetzt. Die Federbeine 6 weisen an ihren unteren (inneren) Enden außenseitige Rastnocken 13 auf. Die radiale Vorspannung oder Abschrägung der Federbeine 6 ist so getroffen, daß die Federbeinhülse mit dem Kranz der Federbeine 6 problemlos in den inneren Bohrlochbereich mit verengtem Durchmesser 2' eingeschoben werden kann. Der obere (äußere) Bereich der Federbeinhülse 4 springt flanschartig radial nach außen und dieser verbreiterte Bereich, als Abstufung 5 bezeichnet, besitzt einen Durchmesser, de rauf den Durchmesser 2'' im oberen (äußeren) Bereich des Lochs 2 angepaßt ist. Auf die Federbeinhülse 4 ist von der Unterseite he ein Federelement 8 insbesondere in Form eines Kautschukrings (aus Silikongummi, Neopren oder dergleichen) aufgeschoben und liegt gegen die untere umlaufende Schulter der verbreiterten Abstufung 5 der Federbeinhülse 4 an. Außerdem weist die Federbeinhülse 4 im Bereich der Abstufung 5 eine umlaufende Innennut 7 auf, die zur Halterung eines (nicht gezeigten) Greifwerkzeugs zum Einführen und Auswechseln der Federbeinhülse 4 dient.

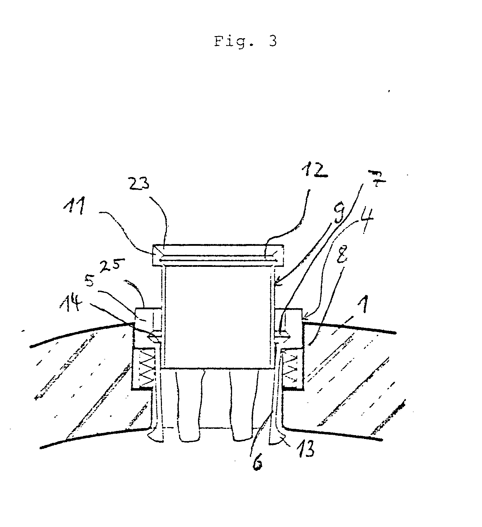

Ist die Federbeinhülse 4 - wi die Fig. 3 zeigt - ganz in die Bohrung 2 eingeschoben, so daß das Federelement 8 gegen die Bohrschulter 3 anliegt und so,daß die Rastnocken 13 knapp unterhalb der unteren Innenkante des Bohrlochs 2 zu stehen kommen, so wird jetzt eine relativ dünnwandige Spreizhülse 9 in die Federbeinhülse 4 eingeschoben, wobei die Federbeine 6 radial nach außen gedrückt werden, so daß die Rastnocken 13 die umlaufende untere Innenkante des Bohrlochs 2 untergreifen und damit die Federbeinhülse 4 positionsrichtig fixieren. die Spreizhülse 9 weist in ih rem oberen (äußeren) Bereich eine axiale Verbreiterung 11 auf, die an die Innenbohrung der Abstufung 5 der Federbeinhülse 4 angepaßt ist. In diesem verbreiterten Durchmesserbereich ist die Spreizhülse 9 ebenfalls mit einer umlaufenden Innennut 12 zum Erfassen mittels eines Einsetz-und Auswechselwerkzeugs versehen.

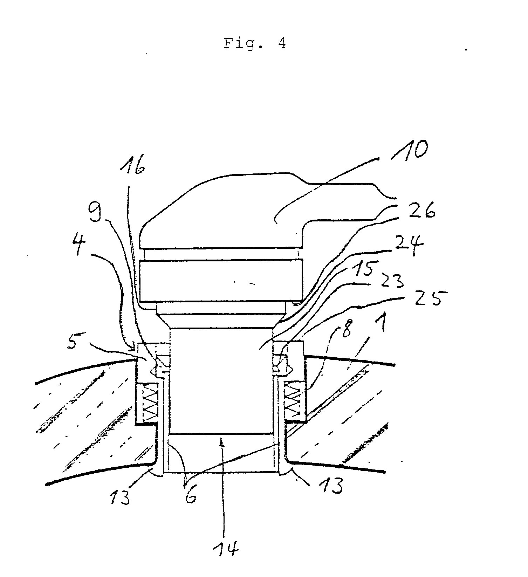

Die Fig. 4 verdeutlicht die vollständig in die Bohrung 2 eingesetzte erfindungsgemäße Applikationsvorrichtung bestehend aus der Federbeinhülse 4 und der Spreizhülse 9 (Doppelhülse) mit der vorteilhaften Ergänzung durch das Federelement 8, welches den Tiefenausgleich für eventuelle Variationen des abgestuften Bohrlochs 2 gewährleistet. Die Fig. 4 läßt gut erkennen, wie jetzt ein Sensor, insbesondere ein Druckaufnehmer 10, mit einer trommelartigen Führungsfläche 15 problemlos und verkantfrei in die Spreizhülse 9 eingeschoben werden kann, wobei die unterseitige (-schädelinnenseitige) Meßflächenmembran 14 des Druckaufnehmers 10 keinerlei unkontrollierten und unerwünschten zusätzlichen Druckkräften und nur den vom Schädelinneren über die Dura ausgehenden Druckkräften ausgesetzt ist. Die richtige Tiefenjustierung ist dadurch gewährleistet, daß sowohl die oberseitige Fläche 23 der Spreizhülse 9 als auch eine abgestufte Paßfläche 24 am Meßaufnehmer 10 mit aufeinander angepaßten Abschrägungen versehen sind. Außerdem sind die obere Umlauffläche 25 der Federbeinhüle 4 als auch eine umlaufende abgestufte Stützfläche 26 am Meßaufnehmer 10 aufeinander angepaßt, so daß die Einschiebtiefe des Meßaufnehmers 10 bei aufeinanderstoßenden Flächen 25, 26 bzw. aufeinanderstoßenden Schrägflächen 23 und 24 genau reproduzierbar gewährleistet ist in bezug auf die Einschiebtiefe sowohl der Federbeinhülse 4 als auch der Spreizhülse 9. Ersichtlicherweise läßt sich der Meßaufnehmer 10 also problemlos einsetzen und auswechseln. Ein Einschrauben oder Einklipsen mittels Bajonettverschluß und alle damit verbundenen Probleme, wie oben erläutert, entfallen.

Sehr entscheidend für ein genaues Meßergebnis ist, daß nicht der Drucksensor 10 beim Einschieben in die Applikationshülsen die Federbeine 6 der Federbeinhülse 4 auseinanderspreizt. Dies hat bereits die Spreizhülse 9 besorgt. Damit ist der Drucksensor 10 gegen unerwünschte Radialkräfte, insbesondere im unteren Kantenbereich der Meßmembran 14, geschützt.

Die Federbeinhülse 4 kann als Kunststoffteil, insbesondere als Kunststoffspritzteil, gefertigt sein. Versuche haben ergeben, daß sich bei Verwendung von Kunststoff-Federbeinhülsen ein günstiges Verhältnis zwischen axialer Vortriebskraft beim Einsetzen der metallischen Spreizhülse 9 in Relation zur Reibung der Federbeine 6 an der Bohrlochwandung 2' ergibt. Daher können mit Kunststoff-Federbeinhülsen größere Stufenstreuungen ausgeglichen werden als mit metallischen Federbeinhülsen. Ein ähnlicher vorteilhafter Effekt ergibt sich dann, wenn bei Verwendung einer Metallegierung für die Federbeinhülse wenigstens die Federbeine 6 kunststoffbeschichtet sind, beispielsweise mit dem unter dem Handelsnamen "Teflon" bekannten Kunststoffmaterial.

Ein wesentlicher Vorteil der Erfindung ergibt sich aus folgendem:
Der Innendurchmesser der Spreizhülse 9 ist geringfügig größer als der Außendurchmesser des Druckaufnehmers 10 im Bereich der trommelartigen Führungsfläche 15. Dadurch erfährt der Druckaufnehmer 10 beim Einführen in die Spreizhülse 9 keinerlei zusätzliche unerwünschte Druckeinwirkung insbesondere im Bereich seiner unteren Umlaufkante, also im Bereich der Meßflächenmembran 14, und zwar auch dann nicht, wenn die dünnwandige Spreizhülse 9 durch das Auseinanderdrücken der Federbeine 6 geringfügig verformt wird. Andererseits wird der genaue Tiefenbezug beim Einschieben des Druckaufnehmers 10 in die Doppelhülsenanordnung 4, 9 im Zusammenwirken der Schrägfläche 23 an der Spreizhülse 9 und der Schrägfläche 24 am Druckaufnehmer 10 gewährleistet, während die axiale Führung und fixierung des Druckaufnehmers 10 in der Doppelhülsenanordnung 4, 9 durch die Paßfläche 16 am Druckaufnehmer 10 im Zusammenwirken mit der umlaufenden Innenkante der radial nach außen springenden Abstufung 5 an der Spreizhülse erreicht wird. Diese axiale Führung und Fixierung des Meßaufnehmers 10 kann auch durch eine oder mehrere Nuten in der Abstufung 5 im Zusammenwirken mit entsprechend angepaßten Vorsprüngen oder Stegen anstelle oder im Bereich der Paßfläche 16 gewährleistet werden. Ein Bajonettverschluß oder dergleichen ist nicht erforderlich.

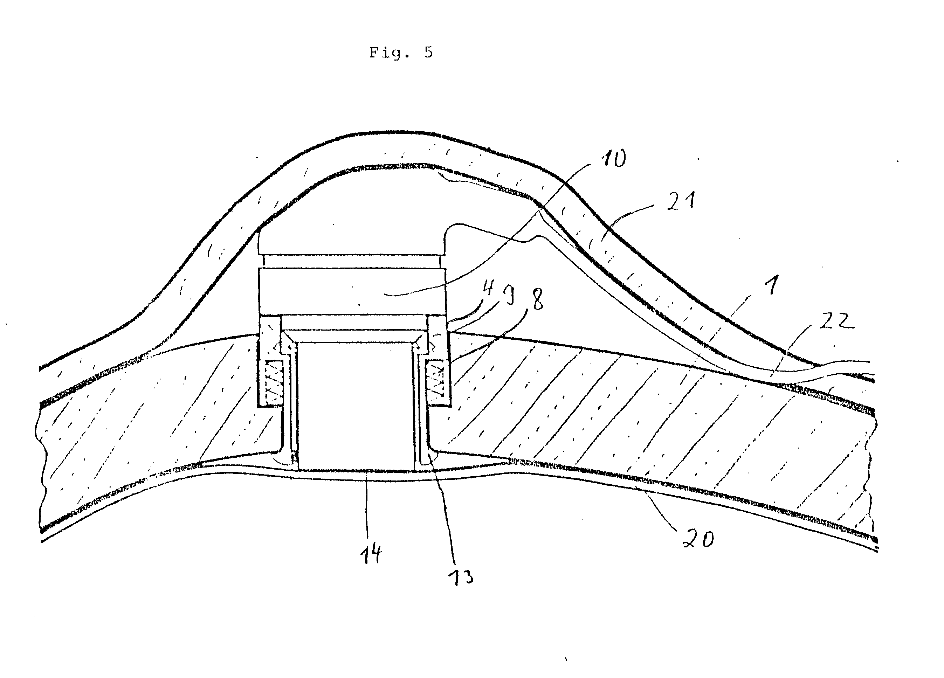

Die Fig. 5 zeigt eine praxisnahe Meßanordnung unter Verwendung der erfindungsgemäßen Applikationsvorrichtung mit Federbeinhülse 4 und Spreizhülse 9. Der Chirurg hat die Kopfschwarte 21 aufgetrennt, mittels Stufentrepan die abgestufte Bohrung 2 eingebracht, sodann die mit Federelement 8 versehene Federbenhülse 4 und anschließend die Spreizhülse 9 eingesetzt, in welche der mit seiner Meßflächenmembran 14 koplanar zur Dura 20 automatisch ausgerichtete Drucksensor 10 eingesteckt wurde. Nach Einbringen einer Durchführung für das Meßlabe; 22 zum Meßgerätwird die Kopfschwarte 21 über die gesamte Meßandordnung gelegt.

Versuche haben gezeigt, daß sich mit der erfindungsgemäßen Applikationsvorrichtung wesentlich einfacher als bisher Drucksensoren für Schädelinnendruckmessung applizieren lassen bei hervorragender Reproduzierbarkeit und Zuverlässigkeit der Meßrbnisse. Die Handhabung und insbesondere der Austausch des Biosensors (Drucksensors) läßt sich auf einfache Weise erreichen. Das ringartige Federelement 8 auf die Federbeinhülse 4 gleicht mit seiner Teleskopfunktion eventuell verbleibende Stufenhöhenstreuungen problemlos aus.

Sofern andere Biosensoren, wie z. B. transdurale oder auch subdurale $pO_2$-oder $pCO_2$-Sensoren mit entsprechender äußerer Gehäuseform verwendet werden, lassen sich diese ebenfalls einfach und reproduzierbar im Schädel applizieren. Entsprechendes gilt für Stoffwechselsensoren, mit denen sich nach Öffnen der Dura der Stoffwechsel im oberen Liquorraum messen läßt. Ebenso können mittels der erfindungsgemäßen Applikationsvorrichtungen Sensoren für Temperatur und andere Meßgrößen auf einfache Weise appliziert werden.

## Ansprüche

1. Applikationsvorrichtung zur asuwechselbaren Implantation von Biosensoren in die Schädelkalotte mit einer in ein abgestuft gebohrtes Loch (2) im Schädelknochen (1) einzusetzenden rohrförmigen Federbeinhülse (4), deren dem Schädelinneren zugewandte Federbeinenden mit außenseitigen Rastnocken (13) versehen sind, welche zur Fixierung der Federbeinhülsen (4) entlang der inneren Umlaufkante des Lochs (2) dienen und deren oberes Ende im Durchmesser nach außen abgestuft (5) und angepaßt auf den größeren Durchmesser (2″) des äußeren Lochbereichs verbreitert ist, **gekennzeichnet durch** eine in die Federbeinhülse (4) mit Gleitsitz einzusteckende Spreizhülse (9), welche die Federbeine (6) der Federbeinhülse (4) derart gegen den unteren verengten Wandbereich (2') des Lochs (2) nach außen spreizt, daß die außenseitigen Rastnocken (13) der Federbeine (6) die innere umlaufende Kante des Lochs (2) untergreifen und deren Innendurchmesser so bemessen ist, daß der Biosensor (10) mit geringem Spiel geführt ist, derart, daß auch bei durch den Radialdruck der Federbeine (6) auftretender geringer Deformation der Spreizhülse (9) ein Verklemmen des Biosensors (10) ausgeschlossen ist.

2. Applikationsvorrichtung nach Anspruch 1, **dadurch gekennzeichnet,** daß die Spreizhülse (9) in ihrem oberen (äußeren) Bereich eine axiale Verbreiterung (11) mit innenseitiger umlaufender Schrägfläche (23) aufweist, die bei Einschieben des Biosensors (10) in die Spreizhülse (9) einen Tiefenbezug für die Einstecktiefe des Biosensors (10) durch Kontaktberührung mit einer radial nach außen abgestuften umlaufenden Schrägfläche (24) am Biosensor (10) festlegt.

3. Applikationsvorrichtung nach Anspruch 1 oder 2, **dadurch gekennzeichnet,** daß die Innenabmessungen der nach außen springenden Abstufung (5) der Federbeinhülse (4) zur paßgenauen Führung und Fixierung des Biosensors (10) in der Meßposition auf eine Paßfläche (16) am Körper des Biosensors (10) angepaßt sind.

4. Applikationsvorrichtung nach einem der vorstehenden Ansprüche, **gekennzeichnet durch** ein ringförmiges Federlement (8) zum axialen Toleranzausgleich der Federbeinhülse (4) auf unterschiedliche Stufenhöhen im Loch (2) und/oder auf unterschiedliche Dicken des Schädelknochens (1), das auf den unteren, dem Schädelinneren zugekehrten Bereich der Federbeinhülse (4) aufgeschoben ist und bei eingesetzter Federbeinhülse (4) einerseits gegen den umlaufenden Stufenrand (3) des Lochs (2) und andererseits gegen die Unterfläche der Abstufung (5) der Federbeinhülse (4) anliegt.

5. Applikationsvorrichtung nach Anspruch 4, **dadurch gekennzeichnet,** daß das Federelement (8) ein Ring aus Silikonkautschuk ist.

6. Applikationsvorrichtung nach Anspruch 1, **dadurch gekennzeichnet,** daß die Spreizhülse (9) mit Greifmitteln (12) zum Einsetzen und Auswechseln mittels eines funktionell angepaßten Werkzeugs versehen ist.

7. Applikationsvorrichtung nach Anspruch 6, **dadurch gekennzeichnet,** daß die axiale Verbreiterung (11) der Spreizhülse (9) innseitig eine umlaufende Nut (12) aufweist zum Erfassen mittels eines in die Spreizhülse (9) einführbaren Greifwerkzeugs.

8. Applikationsvorrichtung nach einem der vorstehen den Ansprüce, **dadurch gekennzeichnet,** daß die Federbeinhülse (4) mit Greifmitteln zum Einsetzen und Auswechseln mittels eines funktionell angepaßten Werkzeugs versehen ist.

9. Applikationsvorrichtung nach Anspruch 8, **dadurch gekennzeichnet,** daß die nach außen springende Abstufung (5) der Federbeinhülse (4) eine innseitig umlaufende Nut (7) aufweist zum Erfassen mittels eines in die Federbeinhülse (4) einführbaren Greifwerkzeugs.

10. Applikationsvorrichtung nach einem der vorstehenden Ansprüche, **dadurch gekennzeichnet,** daß die Federbeinhülse (9) aus Kunststoff besteht.

11. Applikationsvorrichtung nach einem der Ansprüche 1 bis 9, **dadurch gekennzeichnet,** daß die Federbeinhülse (4) aus mindestens im Bereich der Federbeine (6) kunststoffbeschichtetem Metal besteht.

Fig. 1

Fig. 2

Fig. 3

Fig. 4

Fig. 5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y,D | EP-A-0 074 037  (KOSCHKE et al.) <br> * Figuren 1-3; Seite 1, Absätze 2,3; Seite 10, Absätze 2,3; Seiten 11,12 * | 1 | A 61 B  5/03 |
| | --- | | |
| Y | US-A-3 078 064  (TURNBULL) <br> * Figuren 3,4; Spalte 1, Zeilen 28-49 * | 1 | |
| | --- | | |
| A | FR-A-2 046 687  (SOCIETE G.M.T.) <br><br> * Figur 5 * | | |
| | ----- | | |

RECHERCHIERTE SACHGEBIETE (Int. Cl.4)

A 61 B
A 61 F
A 61 M
F 16 B
F 16 L

Der vorliegende Recherchenbericht wurde für alle Patentansprüche erstellt.

| Recherchenort <br> DEN HAAG | Abschlußdatum der Recherche <br> 05-02-1987 | Prüfer <br> FISCHER G.H. |
|---|---|---|